# EUROPÄISCHE PATENTSCHRIFT

(11) **EP 3 541 441 B1**
(45) Veröffentlichungstag und Bekanntmachung des Hinweises auf die Patenterteilung: **10.03.2021**
(21) Anmeldenummer: 17798208.9
(22) Anmeldetag: 15.11.2017
(51) Int. Cl.: A61L 27/36, A61L 2/08, A61L 2/00, A01N 1/02

(54) **VERFAHREN ZUM AUFBEREITEN EINES TRANSPLANTATES**
METHOD FOR PREPARING A GRAFT
PROCÉDÉ POUR PRÉPARER UN TRANSPLANT

(30) Priorität: 16.11.2016 DE 102016121982
(43) Veröffentlichungstag der Anmeldung: 25.09.2019
(73) Patentinhaber: Fraunhofer-Gesellschaft zur Förderung der angewandten Forschung e.V., 80686 München (DE)
(72) Erfinder: WETZEL, Christiane, 01217 Dresden (DE); SCHÖNFELDER, Jessy, 01309 Dresden (DE); WALKER, Simona, 01277 Dresden (DE); KUBUSCH, Jörg, 01279 Dresden (DE)
(86) Internationale Anmeldenummer: PCT/EP2017/079364
(87) Internationale Veröffentlichungsnummer: WO 2018/091553

(56) Entgegenhaltungen:
- US-A1- 2003 068 815

## Beschreibung

In der Humanmedizin gelangen immer häufiger Transplantate tierischen oder menschlichen Ursprungs zum Einsatz, um die Lebenszeitspanne von Menschen zu verlängern und/oder deren Lebensqualität zu erhöhen. Eine Herausforderung beim Aufbereiten derartiger Transplantate vor dem Einsatz der Transplantate in eine Empfängerperson besteht darin, eine Sterilisation des zu transplantierenden Gewebes mit dem Beibehalten der Biofunktionalität und der Biokompatibilität zu kombinieren. Biokompatibel sind Transplantate dann, wenn diese im direkten Kontakt mit lebenden Geweben keinen negativen Einfluss auf deren Stoffwechsel ausüben. Betroffen hiervon sind alle transplantierbaren Gewebe menschlichen oder tierischen Ursprungs. Beispielhaft seien an dieser Stelle Herzklappen, Faszien, Hirnhäute, Sehnen, Bänder, Haut, Blutgefäße, Knochen und Cornea genannt.

Kollagenbasierte Transplantate unterliegen im Körper einem Abbau durch Matrixmetalloproteasen, wie es beispielsweise in Goo; Hwang; Choi; Cho; Suh; Development of collagenase-resistant collagen and its interaction with adult human dermal fibroblasts, Biomaterials 24, 2003, pp. 5099-5113, beschrieben ist. Durch den gezielten Einsatz von Vernetzungsmitteln kann die Degradationsrate beeinflusst werden. In Arcidiacono; Corvi; Severiist, Functional analysis of bioprosthetic heart valves, Journal of Biomechanics 38, 2005, pp.1483-1490, ist offenbart, dass Perikardgewebe durch den Einsatz von toxischem Glutaraldehyd auf chemischem Wege vernetzt und dann als Material für biologische Herzklappenprothesen verwendet werden kann. Dabei verringert die Glutaraldehyd-Vernetzung gleichzeitig auch die Antigenität des Gewebes, erhöht die mechanische Stabilität und wirkt desinfizierend. Trotz dieser Vorteile gibt es auch Veröffentlichungen, die bei derartiger Vorgehensweise auf eine beschleunigte Kalzifizierung des Materials durch die Glutaraldehyd-Vernetzung verweisen (Gendler; Nimni, Toxic reactions evoked by glutaraldehyde-fixed pericardium and cardiac valve tissue bioprosthesis, Journal of Biomedical Materials Research, Vol. 18, 1984, pp. 727-736).
Durch Kalzifizierung und Degradation wird die Funktionsdauer der Klappen stark eingeschränkt. Die durchschnittliche Zeit bis zum Ausfall einer Glutaraldehyd-vernetzten Klappenprothese beträgt in Abhängigkeit vom Alter eines Patienten 10 bis 15 Jahre.

Als Alternative zum chemischen Vernetzen ist das physikalische Modifizieren von Transplantatgewebe bekannt. Eine Vernetzung von Kollagen wird zum Beispiel durch eine dehydrothermale Behandlung in einem Vakuumofen bei 110 °C für 3 bis 5 Tage erreicht (Weadock; Olson; Silver, Evaluation of Collagen Crosslinking Techniques, Biomaterials, Medical Devices, and Artificial Organs, Vol. 11, Nr.4, 1983, pp. 293-318). Vorteile dieser Methode bestehen darin, dass diese keine zytotoxischen Nebenwirkungen hervorruft und eine ausreichend hohe Zugfestigkeit des behandelten Kollagens bewirkt. Nachteilig wirkt sich hingegen die lange Behandlungsdauer von einigen Tagen sowie eine Denaturierung und Degradation des Kollagens aus.

Eine weitere Vernetzungsmethode ist die Behandlung von Kollagenfasern mit UV-Strahlung, die meist nur eine oberflächlich stattfindende Modifizierung bewirkt, Weadock, Miller, Bellincampi, Zawadsky, Dunn, Physical crosslinking of collagen fibers: Comparison of ultraviolet irradiation and dehydrothermal treatment, Journal of Biomedical Materials Research, Vol. 29, 1995, pp. 1373-1379. Nachteilig ist hingegen der Sachverhalt, dass auch dieses Verfahren zur Degradation führt.

Es ist ebenfalls bekannt, kollagenhaltige Materialien mittels Gammastrahlen zu modifizieren. Gammastrahlen haben im Gegensatz zu UV-Strahlen eine sehr hohe Reichweite (da Silva Aquino, Sterilization by Gamma Irradiation, Gamma Radiation, 2012, pp. 171-206) und durchstrahlen damit in der Regel das gesamte Substrat inklusive der Verpackung. Neben einer erwünschten Vernetzung des Kollagens und einer Sterilisation des Substrates geht dieses Verfahren mit starken Degradations- und Denaturierungserscheinungen im Material einher.

In US 6 203 755 B1 ist das Sterilisieren biologischen Gewebes mit hochenergetischen Elektronenstrahlen beschrieben. Hierbei gelangen beschleunigte Elektronen mit einer Energie im MeV-Bereich zur Anwendung. Nachteilig wirken sich bei dieser Vorgehensweise Veränderungen in der Konfiguration der kollagenen Polypeptidketten aus.

Auch sind Kombinationen der zuvor beschriebenen Einzelverfahren bekannt. So beschreibt zum Beispiel US 2003/0068815 A1 eine Verfahren, bei welchem das Kollagen in einem Transplantat aus einem tierischen Gewebe mittels chemischer Substanzen vernetzt und das Transplantat zum Beispiel mittels hochenergetischer Elektronenstrahlen sterilisiert wird.

Nachteilig wirkt sich auch hierbei aus, dass mittels chemischer Vernetzung nur ein beschränkter Vernetzungsgrad erzielbar ist und dass das Sterilisieren mittels hochenergetischen Elektronenstrahlen mit einer Reduzierung der Biokompatibilität einhergeht.

Der Erfindung liegt daher das technische Problem zugrunde, ein Verfahren zum Aufbereiten von Transplantaten tierischen oder menschlichen Ursprungs zu schaffen, mittels dessen die Nachteile aus dem Stand der Technik überwunden werden können. Insbesondere soll es mit dem erfindungsgemäßen Verfahren auch möglich sein, eine gute Vernetzung des Kollagens in Transplantaten und das Sterilisieren von Transplantaten ohne den Einsatz toxischer Substanzen zu erzielen und dabei die Biokompatibilität der Transplantate beizubehalten.

Die Lösung des technischen Problems ergibt sich durch Gegenstände mit den Merkmalen des Patentanspruchs 1. Weitere vorteilhafte Ausgestaltungen der Erfindung ergeben sich aus den abhängigen Ansprüchen.

Beim erfindungsgemäßen Verfahren zum Aufbereiten eines aus einem tierischen oder humanen Gewebe gewonnenen Transplantates, wird das Transplantat zunächst in einer ersten Flüssigkeit deponiert, wobei in der ersten Flüssigkeit zumindest eine Substanz enthalten ist, die das Vernetzen von Kollagen initiiert und/oder aktiviert. Als derartige Substanzen können beispielsweise ein Enzym, eine Aminosäure und/oder ein Zucker verwendet werden. Besonders vorteilhaft ist es für das erfindungsgemäße Verfahren, wenn ein Photoinitiator als Substanz verwendet wird, die das Vernetzen von Kollagen initiiert und/oder aktiviert. Unter einem Photoinitiator ist eine chemische Verbindung zu verstehen, die nach Absorption von ultraviolettem Licht in einer Photolysereaktion zerfällt und dabei reaktive Spezies bildet, die wiederum eine Reaktion initiieren oder aktivieren können. Als Photoinitiator kann beispielsweise ein Vitamin, wie zum Beispiel Riboflavin, verwendet werden.

Das Deponieren des Transplantates in der ersten Flüssigkeit, bei welchem das Transplantat von der ersten Flüssigkeit und dem darin enthaltenen Photoinitiator durchtränkt werden soll, kann in Abhängigkeit von der Art des Transplantates eine Zeitspanne von einigen Minuten bis hin zu mehreren Tagen umfassen. Welche Zeitspanne bei einer konkreten Aufgabenstellung zweckmäßig ist, lässt sich mit Laborversuchen ermitteln.

Nach dem Verstreichen der Zeit für das Durchtränken des Transplantates mit der ersten Flüssigkeit wird das Transplantat aus der ersten Flüssigkeit entfernt und noch im mit der ersten Flüssigkeit befeuchteten Zustand, möglichst die gesamte Oberfläche des Transplantates mit elektromagnetischer Strahlung im Wellenlängenbereich von 310 nm bis 450 nm, das heißt mit ultravioletter Strahlung beaufschlagt, um eine Vernetzung des Kollagens im Transplantat zu bewirken. Die in der ersten Flüssigkeit enthaltene Substanz, die beim Deponieren in der ersten Flüssigkeit vom Transplantat aufgenommen wurde, wirkt hierbei unterstützend. Das vollflächige Beaufschlagen des Transplantates mit ultravioletter (UV) Strahlung kann beispielsweise realisiert werden, indem das Transplantat mit mehreren UV-Strahlungsquellen vollumfänglich, gleichzeitig bestrahlt wird oder indem die verschiedenen Oberflächenbereiche des Transplantates mit einer oder mehreren UV-Strahlungsquellen nacheinander mit ultravioletter Strahlung beaufschlagt werden. Bei einer Ausführungsform des erfindungsgemäßen Verfahrens wird das Beaufschlagen des Transplantates mit ultravioletter Strahlung mit einer Dosis von 100 mJ/cm² bis 2500 mJ/cm² durchgeführt.

Das erfindungsgemäße Verfahren umfasst ferner den Verfahrensschritt des Beaufschlagens der gesamten Oberfläche des Transplantates mit beschleunigten, niederenergetischen Elektronen mit einer Energie von 100 keV bis 500 keV, welcher nach dem Beaufschlagen des Transplantates mit ultravioletter Strahlung durchgeführt wird. Durch das Beaufschlagen des Transplantates mit beschleunigten, niederenergetischen Elektronen wird das Transplantat zum einen sterilisiert, zum anderen bewirkt das Beaufschlagen mit beschleunigten, niederenergetischen Elektronen eine Erhöhung des Vernetzungsgrades des Kollagens im Transplantat.

Bei einer weiteren Ausführungsform wird das Transplantat zwischen dem Beaufschlagen mit ultravioletter Strahlung und dem Beaufschlagen mit beschleunigten, niederenergetischen Elektronen in einer zweiten Flüssigkeit deponiert. Das ist insbesondere dann vorteilhaft, wenn zwischen dem Beaufschlagen des Substrates mit ultravioletter Strahlung und dem Beaufschlagen des Substrates mit beschleunigten, niederenergetischen Elektronen eine größere Zeitspanne vorgesehen ist, welche mit dem Austrocknen des Transplantates einhergeht. Hierbei ist es vorteilhaft, wenn in der zweiten Flüssigkeit ebenfalls eine Substanz enthalten ist, die das Vernetzen von Kollagen initiiert und/oder aktiviert, wodurch das weitere Vernetzen des Kollagens im Transplantat während des Beaufschlagens mit beschleunigten, niederenergetischen Elektronen unterstützt wird.

Bei einer weiteren Option wird das Transplantat erst mit beschleunigten, niederenergetischen Elektronen beaufschlagt, nachdem das Transplantat in eine Verpackung eingebracht wurde, die beispielsweise aus einem Kunststoff, einem Metall oder aus Glas bestehen kann.

Die vorliegende Erfindung wird nachfolgend anhand eines Ausführungsbeispiels näher erläutert. Das Perikardgewebe eines Schweins soll als Transplantat aufbereitet werden. Hierzu werden zunächst mit bekannten Verfahrensschritten angrenzende Gewebereste vom Perikardgewebe sowie alle zellulären Bestandteile des Perikardgewebes entfernt. Die nach dieser Behandlung übrig bleibenden Bestandteile des Perikardgewebes werden nachfolgend als Transplantat bezeichnet.

Erfindungsgemäß wird das Transplantat für eine Zeitspanne von 20 Stunden in einer Riboflavinlösung mit einer Konzentration von 260 µmol/l deponiert, welche 2% Dextran T500 enthält, um das Transplantat mit dem Photoinitiator Riboflavin zu durchtränken. Wird beim erfindungsgemäßen Verfahren Riboflavin als Photoinitiator verwendet, sind beispielsweise Riboflavinlösungen mit einer Konzentration von 0,1 µmol/l bis 300 mmol/l als erste Flüssigkeit geeignet. Danach wird das Transplantat aus der Riboflavinlösung entfernt und das noch mit der Riboflavinlösung befeuchtete flächige Transplantat von beiden Seiten für jeweils 30 Minuten mit ultravioletter Strahlung mit einer Leistungsdichte von 0,3 mW/cm² beaufschlagt, um eine Vernetzung des Kollagens im Transplantat zu bewirken.

Ein weiteres Vernetzen des Kollagens im Transplantat sowie das Sterilisieren des Transplantates erfolgt erfindungsgemäß, indem das Transplantat nach dem Behandeln mit UV-Strahlung in einer Arbeitskammer beidseitig mit beschleunigten, niederenergetischen Elektronen beaufschlagt wird. Damit die Biofunktionalität des Transplantates erhalten bleibt, gelangen beim erfindungsgemäßen Verfahren lediglich niederenergetische Elektronen zur Anwendung. Hierbei werden beschleunigte Elektronen mit einer Energie von 100 keV bis 500 keV verwendet und das Beaufschlagen eines Transplantates mit niederenergetischen Elektronen erfolgt erfindungsgemäß mit einer Dosis von 1 kGy bis 500 kGy.

Beim Ausführungsbeispiel wird in der Arbeitskammer Atmosphärendruck angewendet und die in der Arbeitskammer befindliche Luft mit Stickstoff angereichert, bis ein Sauerstoffanteil von maximal 100 ppm eingestellt ist. Alternativ können erfindungsgemäß beim Beaufschlagen eines Transplantates mit beschleunigten, niederenergetischen Elektronen auch andere Druckverhältnisse in der Arbeitskammer eingestellt und in die Arbeitskammer auch andere Gase oder Gasgemische eingelassen werden. Geeignet für das erfindungsgemäße Verfahren sind zum Beispiel Luft, Helium, Argon, Kohlendioxid, Kohlenmonoxid, Stickstoffmonoxid, Sauerstoff, Neon, Methan, Krypton, Wasserstoff, oder Gemische mindestens zweier zuvor genannter Komponenten.

Das erfindungsgemäße Verfahren wurde lediglich bespielhaft anhand eines Perikardgewebes beschrieben, ist jedoch nicht auf diese Gewebeart beschränkt. Vielmehr ist das erfindungsgemäße Verfahren für alle transplantierbaren Gewebearten tierischen und menschlichen Ursprungs geeignet. Bespielhaft seien an dieser Stelle aufgeführt Herzklappen-, Faszien-, Hirnhaut-, Sehnen-, Bänder-, Haut-, Blutgefäß-, Knochen- und Cornea-Gewebe.

Bei Anwendung des erfindungsgemäßen Verfahrens auf Transplantatgewebe verschiedener Gewebearten konnte der experimentelle Nachweis erbracht werden, dass eine sehr gute Vernetzung des Kollagens im Transplantat und das Sterilisieren des Transplantates auch ohne den Einsatz toxischer Substanzen und bei Beibehaltung der Biokompatibilität erzielbar ist. Bei erfindungsgemäß aufbereitetem Transplantatgewebe konnte lediglich eine geringe bis gar keine Kalzifizierung des Gewebes festgestellt werden, da die kalzifizierungsbegünstigende Substanz Glutaraldehyd beim erfindungsgemäßen Verfahren nicht zum Einsatz gelangt. Deshalb ist von einer längeren Haltbarkeit der Transplantate auszugehen.

## Patentansprüche

1. Verfahren zum Aufbereiten eines Transplantates, welches aus einem tierischen oder humanen Gewebe gewonnen wurde, **gekennzeichnet durch** folgende Verfahrensschritte,
a) Deponieren des Transplantates in einer ersten Flüssigkeit, welche eine Substanz enthält, die das Vernetzen von Kollagen initiiert und/oder aktiviert;
b) Entfernen des Transplantates aus der ersten Flüssigkeit und Beaufschlagen des mit der ersten Flüssigkeit befeuchteten Transplantates mit ultravioletter Strahlung;
c) Beaufschlagen des Transplantates mit beschleunigten, niederenergetischen Elektronen, wobei beschleunigte Elektronen mit einer Energie von 100 keV bis 500 keV verwendet werden.

2. Verfahren nach Anspruch 1, **dadurch gekennzeichnet, dass** ein Enzym, eine Aminosäure und/oder ein Zucker als Substanz verwendet werden, welche das Vernetzen von Kollagen initiiert und/oder aktiviert.

3. Verfahren nach Anspruch 1, **dadurch gekennzeichnet, dass** ein Photoinitiator als Substanz verwendet wird, welche das Vernetzen von Kollagen initiiert und/oder aktiviert.

4. Verfahren nach Anspruch 3, **dadurch gekennzeichnet, dass** ein Vitamin als Photoinitiator verwendet wird.

5. Verfahren nach einem der vorhergehenden Ansprüche, **dadurch gekennzeichnet, dass** das Beaufschlagen des Transplantates mit ultravioletter Strahlung auf der gesamten Oberfläche des Transplantates durchgeführt wird.

6. Verfahren nach einem der vorhergehenden Ansprüche, **dadurch gekennzeichnet, dass** das Beaufschlagen des Transplantates mit ultravioletter Strahlung mit einer Dosis von 100 mJ/cm² bis 2500 mJ/cm² durchgeführt wird.

7. Verfahren nach einem der vorhergehenden Ansprüche, **dadurch gekennzeichnet, dass** das Beaufschlagen des Oberflächenbereiches des Substrates mit niederenergetischen Elektronen mit einer Dosis von 1 kGy bis 500 kGy erfolgt.

8. Verfahren nach einem der vorhergehenden Ansprüche, **dadurch gekennzeichnet, dass** das Transplantat zwischen dem Beaufschlagen mit ultravioletter Strahlung und dem Beaufschlagen mit beschleunigten, niederenergetischen Elektronen in einer zweiten Flüssigkeit deponiert wird.

9. Verfahren nach Anspruch 8, **dadurch gekennzeichnet, dass** eine zweite Flüssigkeit verwendet wird, welche eine Substanz enthält, die das Vernetzen von Kollagen initiiert und/oder aktiviert.

## Claims

1. Method for processing a transplant which was obtained from an animal or human tissue, **characterized by** the following method steps,
a) depositing the transplant in a first liquid which contains a substance which initiates and/or activates the crosslinking of collagen;
b) removing the transplant from the first liquid and bombarding the transplant moistened with the first liquid with ultraviolet radiation;
c) bombarding the transplant with accelerated, low-energy electrons, wherein accelerated electrons having an energy of 100 keV to 500 keV are used.

2. Method according to Claim 1, **characterized in that** an enzyme, an amino acid and/or a sugar are used as substance which initiates and/or activates the crosslinking of collagen.

3. Method according to Claim 1, **characterized in that** a photoinitiator is used as substance which initiates and/or activates the crosslinking of collagen.

4. Method according to Claim 3, **characterized in that** a vitamin is used as photoinitiator.

5. Method according to any of the preceding claims, **characterized in that** the bombardment of the transplant with ultraviolet radiation is carried out on the entire surface of the transplant.

6. Method according to any of the preceding claims, **characterized in that** the bombardment of the transplant with ultraviolet radiation is carried out with a dose of 100 mJ/cm² to 2500 mJ/cm².

7. Method according to any of the preceding claims, **characterized in that** the bombardment of the surface region of the transplant with low-energy electrons is carried out with a dose of 1 kGy to 500 kGy.

8. Method according to any of the preceding claims, **characterized in that** the transplant is deposited in a second liquid between the bombardment with ultraviolet radiation and the bombardment with accelerated, low-energy electrons.

9. Method according to Claim 8, **characterized in that** a second liquid is used which contains a substance which initiates and/or activates the crosslinking of collagen.

## Revendications

1. Procédé de préparation d'un greffon, lequel a été obtenu à partir d'un tissu animal ou humain, **caractérisé par** les étapes suivantes :
a) dépôt du greffon dans un premier liquide qui contient une substance, laquelle initie et/ou active la réticulation du collagène ;
b) retrait du greffon du premier liquide et exposition à un rayonnement ultraviolet du greffon humidifié avec le premier liquide ;
c) exposition du greffon à des électrons accélérés à faible énergie, des électrons accélérés ayant une énergie de 100 keV à 500 keV étant utilisés.

2. Procédé selon la revendication 1, **caractérisé en ce qu'**un enzyme, un acide aminé et/ou un sucre est utilisé en tant que substance qui initie et/ou active la réticulation du collagène.

3. Procédé selon la revendication 1, **caractérisé en ce qu'**un photo-initiateur est utilisé en tant que substance qui initie et/ou active la réticulation du collagène.

4. Procédé selon la revendication 3, **caractérisé en ce qu'**une vitamine est utilisée en tant que photo-initiateur.

5. Procédé selon l'une des revendications précédentes, **caractérisé en ce que** l'exposition du greffon à un rayonnement ultraviolet est effectuée sur la totalité de la surface du greffon.

6. Procédé selon l'une des revendications précédentes, **caractérisé en ce que** l'exposition du greffon à un rayonnement ultraviolet est effectuée avec une dose de 100 mJ/cm² à 2500 mJ/cm².

7. Procédé selon l'une des revendications précédentes, **caractérisé en ce que** l'exposition de la zone de la surface du greffon à des électrons à faible énergie est effectuée avec une dose de 1 kGy à 500 kGy.

8. Procédé selon l'une des revendications précédentes, **caractérisé en ce que** le greffon, entre l'exposition au rayonnement ultraviolet et l'exposition aux électrons accélérés à faible énergie, est déposé dans un deuxième liquide.

9. Procédé selon la revendication 8, **caractérisé en ce qu'**un deuxième liquide est utilisé, qui contient une substance qui initie et/ou active la réticulation du collagène.
